# FASCICULE DE BREVET EUROPEEN

(11) **EP 1 637 095 B1**
(45) Date de publication et mention de la délivrance du brevet: **21.03.2012**
(21) Numéro de dépôt: 05291953.7
(22) Date de dépôt: 20.09.2005
(51) Int. Cl.: A61F 2/30

(54) **Implant articulaire d'interposition**
Zwischenlegungsgelenkimplantat
Interposition joint implant

(30) Priorité: 21.09.2004 FR 0409955
(43) Date de publication de la demande: 22.03.2006
(73) Titulaire: Newdeal, 69800 Saint-Priest (FR)
(72) Inventeur: Ragusa, Mathieu Antoine Joseph, 38240 Meylan (FR); Diebold, Patrice François, 54000 Nancy (FR)
(74) Mandataire: Martin, Didier Roland Valéry

(56) Documents cités:
- EP-A- 1 112 753
- WO-A-02/49547
- WO-A-98/47449
- WO-A-02/054992
- WO-A-03/061522
- DE-C- 4 140 838
- DE-U- 20 003 360
- FR-A- 2 470 583
- FR-A- 2 803 191
- US-A- 4 936 860
- US-B1- 6 290 726

## Description

La présente invention se rapporte au domaine technique des implants articulaires d'interposition destinés à être implantés, temporairement ou de façon permanente, à l'interface articulaire entre deux os, par exemple un métatarsien et une phalange.

La présente invention concerne plus particulièrement un implant articulaire d'interposition destiné à être mis en place entre les surfaces articulaires d'au moins deux os séparés par une interface articulaire, en vue de réaliser l'arthroplastie d'une articulation.

En l'occurrence, il s'agit de permettre au patient de recouvrer la mobilité de ladite articulation et, conjointement, de supprimer ou d'atténuer la douleur liée à la dégradation des tissus et/ou à l'inflammation de la zone articulaire.

Il existe plusieurs catégories de prothèses articulaires, couramment utilisées en chirurgie orthopédique et destinées à remplacer les surfaces articulaires altérées.

La première catégorie de prothèse est généralement formée par deux implants fixés chacun respectivement à l'un des os de l'articulation, les implants étant montés mobile l'un par rapport à l'autre de manière à permettre la mobilité de l'articulation. De telles prothèses sont généralement des prothèses permanentes en raison de leur nécessaire fixation aux tissus osseux.

Il existe également une catégorie d'implants à fixation temporaire, constitués par une cupule percée en son centre d'un orifice, et destinés à être mis en place entre deux os, tels qu'un métatarsien et une phalange, grâce à une broche de fixation provisoire de la cupule. Par rapport aux prothèses formées de deux implants décrites précédemment, les implants articulaires à fixation temporaire présentent l'avantage de pouvoir être facilement retirés une fois les tissus fibreux reconstitués, grâce notamment à l'absence d'ancrage définitif de ces implants dans les tissus osseux.

Grâce à la broche de fixation provisoire, ces implants peuvent en outre être centrés et positionnés de façon précise dans l'interface articulaire.

Malgré tous ces avantages, ces implants souffrent néanmoins d'inconvénients non négligeables, liés notamment à l'utilisation de la broche de fixation temporaire.

Tout d'abord, de tels implants nécessitent, pour leur mise en place, d'effectuer une incision suffisamment large pour permettre un centrage correct de la broche. Dans le cas d'une articulation métatarso-phalangienne du pied, la broche est ainsi généralement introduite en premier lieu dans le canal médullaire des phalanges successives, en se dirigeant vers la partie distale de l'orteil, et en second lieu dans le canal médullaire du métatarsien par une technique de va-et-vient. Toutes ces manipulations nécessitent de pratiquer une incision relativement importante et souvent bien supérieure à la taille de l'implant. Or, il est souhaitable, tant du point de vue esthétique que du point de vue des risques d'infection et de douleurs post-opératoires, de réduire la taille des incisions réalisées.

Par ailleurs, la mise en place des implants de l'art antérieur a pour inconvénient de conduire assez fréquemment à un endommagement de la capsule articulaire, notamment lors de la mise en place de la broche de fixation par la technique du va-et-vient.

Enfin, la présence de la broche nécessite généralement une nouvelle intervention chirurgicale en vue de la retirer, ce qui complique encore davantage les suites de l'opération.

Les implants de l'art antérieur nécessitent donc, pour leur mise en place, une intervention relativement lourde, susceptible d'entraîner plusieurs complications non négligeables, et dont les suites sont parfois mal supportées par le patient.

Par ailleurs, les implants de l'art antérieur conservent généralement, malgré la présence de la broche de fixation, une certaine mobilité au sein de l'interface articulaire. En particulier, les implants maintenus à l'aide d'une broche de fixation centrale conservent la possibilité de tourner sur eux-même autour de l'axe longitudinal de la broche. Or, cette mobilité de l'implant est non seulement susceptible d'entraîner une sensation de gêne pour le patient, mais également de ralentir la régénération des tissus osseux et du cartilage. En outre, si l'implant est mal positionné, il peut se produire une érosion anormale des surfaces articulaires adjacentes. Ce phénomène s'observe en particulier dans les articulations métatarso-phalangiennes du pied en raison des contraintes importantes qui y sont exercées.

On connaît par ailleurs, par le document WO-03/061522, un implant articulaire selon le préamble de la revendication 1.

Les objets assignés à l'invention visent par conséquent à proposer un nouvel implant articulaire d'interposition ne présentant pas les inconvénients énumérés précédemment et dont la mise en place et le positionnement au sein de l'interface articulaire sont particulièrement simples et rapides

Un autre objet de l'invention vise à proposer un nouvel implant articulaire d'interposition qui ne nécessite qu'une petite incision pour sa mise en place.

Un autre objet de l'invention vise à proposer un nouvel implant articulaire d'interposition dont le centrage et le positionnement dans l'interface articulaire sont facilités.

Un autre objet de l'invention vise à proposer un nouvel implant articulaire d'interposition qui soit particulièrement stable au sein de l'interface articulaire.

Un autre objet de l'invention vise à proposer un nouvel implant articulaire d'interposition dont la probabilité de migration hors de sa zone d'efficacité au sein de l'interface articulaire est particulièrement faible.

Un autre objet de l'invention vise à proposer un nouvel implant articulaire d'interposition ne nécessitant qu'un nombre minimum d'étapes pour sa mise en place.

Un autre objet de l'invention vise à proposer un nouvel implant articulaire d'interposition qui soit particulièrement simple à fabriquer.

Un autre objet de l'invention vise à proposer un nouvel implant articulaire d'interposition facilitant la régénération des tissus osseux.

Un autre objet de l'invention vise à proposer un nouvel implant articulaire d'interposition adapté à la morphologie de l'articulation métatarso-phalangienne.

Les objets assignés à l'invention sont atteints à l'aide d'un implant articulaire d'interposition selon la revendication 1.

D'autres objets et avantages de l'invention apparaîtront mieux à la lecture de la description qui suit, ainsi qu'à l'aide des dessins annexés, donnés à titre purement illustratif et non limitatif, dans lesquels :
- La figure 1 illustre un implant articulaire d'interposition conforme à l'invention mis en place entre une phalange et un métatarsien.
- La figure 2 illustre, selon une vue de profil, l'implant articulaire d'interposition conforme à l'invention.
- La figure 3, illustre, selon une vue en perspective, l'implant articulaire d'interposition conforme à l'invention.
- La figure 4 illustre, selon une vue de dessus, une variante de réalisation de l'implant articulaire d'interposition conforme à l'invention.
- La figure 5 illustre, selon une vue de dessous, l'implant articulaire d'interposition conforme à l'invention.
- Les figures 6 et 7 illustrent, selon une vue de côté, deux variantes de réalisation de l'implant articulaire d'interposition conforme à l'invention.
- Les figures 8 et 9 illustrent, selon une vue de dessus, deux variantes de réalisation de l'implant articulaire d'interposition conforme à l'invention.

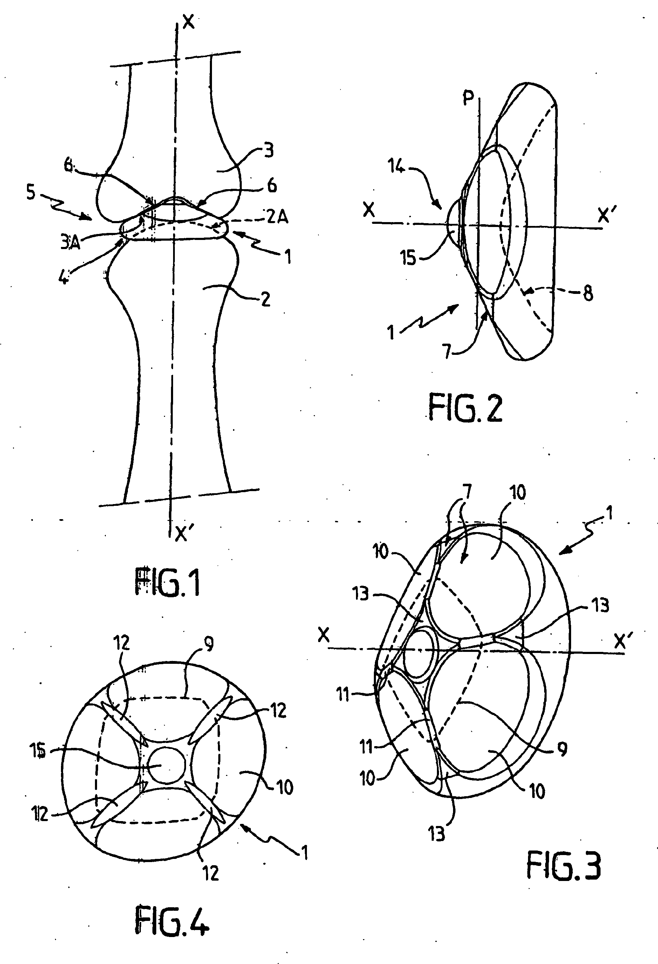

La figure 1 illustre une articulation 5 formée par deux os, par exemple un premier os 2 et un deuxième os 3, et un implant 1 articulaire d'interposition disposé à l'interface entre les deux os 2, 3. Le premier os 2 peut ainsi, par exemple, être formé par un métatarsien, le deuxième os 3 étant alors, par exemple, formé par une phalange. Dans ce cas, l'implant 1 constitue avantageusement un implant métatarso-phalangien. Toutefois, l'articulation 5 pourrait bien évidemment comporter un troisième os, par exemple situé entre le premier os 2 et le deuxième os 3, et ce sans sortir du cadre de l'invention. L'implant 1 articulaire d'interposition conforme à l'invention est ainsi destiné à être mis en place entre les surfaces articulaires 2A, 3A des os 2, 3.

Plus précisément, l'implant 1 articulaire d'interposition est adapté pour être introduit au sein de l'interface articulaire 4 séparant les os 2, 3 afin de réaliser l'arthroplastie de l'articulation 5. Une arthroplastie consiste ainsi à intervenir de façon chirurgicale au niveau d'une articulation en vue de lui restituer sa mobilité. Une telle opération est notamment préconisée dans le cas où le patient souffre d'une affection chronique dégénérative des articulations, telle que l'arthrose.

Au sens de l'invention, le terme *« implant »* fait référence à une pièce de préférence unique et monobloc, destinée à être implantée au sein de l'articulation en vue d'améliorer sa mobilité, et se différencie ainsi des prothèses, et notamment des prothèses articulées constituées de deux pièces, par exemple un implant métatarsien et un implant phalangien montés mobile l'un par rapport à l'autre et fixés respectivement à l'extrémité des os de l'articulation.

L'expression *« implant articulaire d'interposition »* fait ainsi référence à un implant de type charnière, situé à l'interface entre deux os de manière à leur restituer leur mobilité relative.

Afin de permettre et également de faciliter la reconstruction osseuse, il est souvent nécessaire d'immobiliser sensiblement l'implant 1, au moins temporairement, au sein de l'articulation 5. A cet effet, et selon une caractéristique essentielle de l'invention, l'implant 1 conforme à l'invention comporte des moyens d'immobilisation 6, conformés de telle sorte que lorsque l'implant 1 est disposé dans l'interface articulaire 4, les moyens d'immobilisation 6 viennent en appui contre au moins l'une des surfaces articulaires 2A, 3A. Les moyens d'immobilisation 6 assurent ainsi, par friction et adhérence sur la surface articulaire 3A, l'immobilisation automatique de l'implant 1 vis-à-vis de ladite surface articulaire 3A, et ce sans avoir recours à une broche d'ancrage ou à tout autre élément de fixation temporaire ou permanent.

Cette immobilisation spontanée concourt à l'encastrement progressif de l'implant en facilitant la régénération tissulaire qui tend au scellement de celui-ci contre la surface articulaire 3A.

L'intervention chirurgicale peut comprendre une étape de préparation des surfaces articulaires, notamment de la surface 3A, voire de ménagement d'un logement, notamment dans l'os 3, pour améliorer l'assise des moyens d'immobilisation 6 et par conséquent la stabilité de l'implant. Toutefois, de façon particulièrement avantageuse, les moyens d'immobilisation 6 viennent naturellement en appui contre la surface articulaire 3A, sans qu'il soit nécessaire de réséquer ladite surface articulaire, par exemple en y ménageant des découpes planes. Les surfaces articulaires 2A, 3A des os 2, 3 étant sensiblement courbes, l'implant 1 articulaire d'interposition conforme à l'invention comporte au moins une première surface d'appui 7, et préférentiellement une première et une deuxième surface d'appui 7, 8 globalement courbes et de préférence sensiblement parallèles l'une par rapport à l'autre. De façon encore plus préférentielle, les première et deuxième surfaces d'appui 7, 8 présentent sensiblement la même courbure. Les première et deuxième surfaces d'appui 7, 8 forment ainsi avantageusement une interface de contact avec les surfaces articulaires 2A, 3A correspondantes.

L'expression *« globalement courbe »* fait référence au fait que les surfaces d'appui 7, 8, bien que susceptibles de comporter, localement, une ou plusieurs portions de surfaces planes, présentent un aspect général courbé, au moins par morceaux.

Avantageusement, la première surface d'appui 7 est ainsi globalement convexe, la deuxième surface d'appui 8 étant globalement concave. L'implant 1 articulaire d'interposition présente ainsi une forme générale de cupule ou de demi-coque, conférant à l'implant 1 une forme anatomique lui permettant de s'adapter à la morphologie naturelle de l'articulation, et notamment à celle de l'articulation métatarso-phalangienne. Grâce à la forme anatomique de l'implant 1, il n'est alors pas nécessaire de réséquer les surfaces articulaires 2A, 3A ou les extrémités des os 2, 3.

Avantageusement, les moyens d'immobilisation 6 sont solidaires de l'implant 1 et encore plus préférentiellement intégrés structurellement à l'implant 1, c'est-à-dire qu'ils ne sont ni rapportés, ni dissociés dudit implant 1. Cette caractéristique permet notamment d'immobiliser automatiquement l'implant 1 au sein de l'interface articulaire 4, dès son introduction, et donc en un nombre minimum d'étapes.

Selon l'invention, l'implant 1 s'étend, longitudinalement, c'est-à-dire selon une direction sensiblement perpendiculaire au plan d'extension radiale de l'implant, suivant un axe longitudinal X-X' qui, lorsque l'implant est en position au sein de l'interface articulaire 4, est sensiblement confondu avec l'axe des canaux médullaires des premier et deuxième os 2, 3, et par exemple du métatarsien et de la phalange correspondante.

Les moyens d'immobilisation 6 sontconformés pour empêcher la rotation de l'implant 1 sur lui-même par rapport à l'axe longitudinal X-X'. Ceci permet notamment de réduire les possibilités de mobilité et de jeu de l'implant 1 au sein de l'articulation 5, susceptibles non seulement d'entraîner une gêne pour le patient, mais également de conduire à une usure progressive des surfaces articulaires 2A, 3A. L'immobilisation en rotation de l'implant 1 permet ainsi de favoriser la régénération des tissus osseux environnants.

Selon une caractéristique particulièrement avantageuse de l'invention, les moyens d'immobilisation 6 sont disposés sur la première surface d'appui 7 de telle sorte que cette dernière ne présente pas une symétrie de révolution par rapport à l'axe longitudinal X-X'. Ainsi, les moyens d'immobilisation 6 sont disposés de telle sorte que la première surface d'appui 7 présente, le long d'au moins une ligne dite parallèle 9 (par analogie avec les parallèles d'une surface sphérique ou hémisphérique définis par rapport à l'équateur) située à l'intersection entre ladite première surface d'appui 7 et au moins un plan P perpendiculaire à l'axe longitudinal X-X', une courbure discontinue. En effet, contrairement aux implants de l'art antérieur, l'implant 1 conforme à l'invention ne présente pas une symétrie de révolution sur sa première surface d'appui 7.

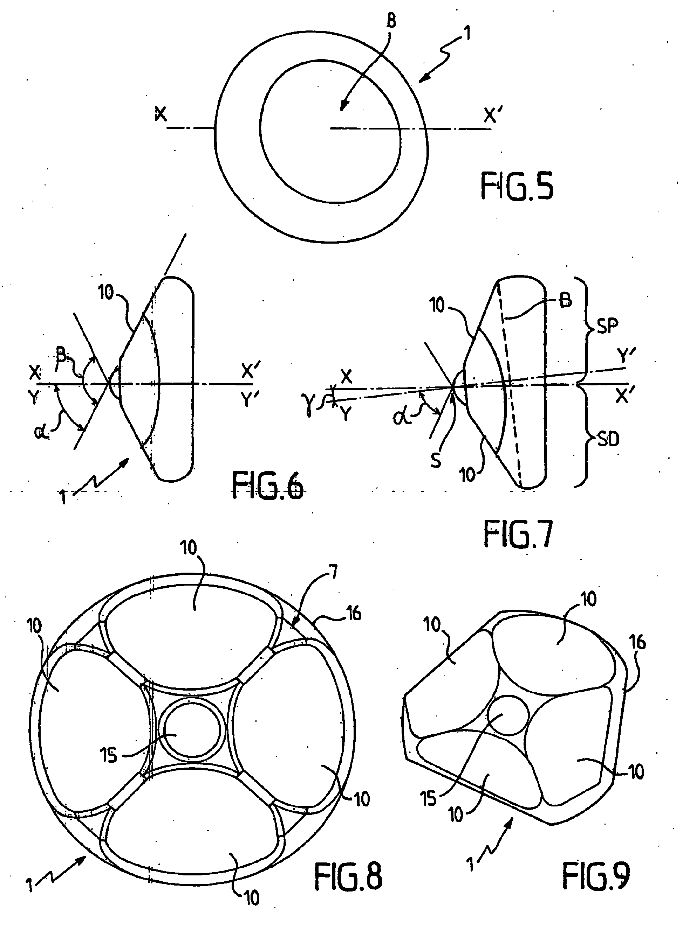

En revanche, la deuxième surface d'appui 8, de préférence concave, de l'implant 1 est avantageusement sensiblement sphérique (figure 5) et présente une symétrie de révolution suivant l'axe longitudinal X-X'. Grâce à sa forme sphérique, la deuxième surface d'appui 8 de l'implant 1 permet la mobilité du premier os 2, suivant une liaison cinématique de type sphérique (ou liaison rotule), à l'interface entre la deuxième surface d'appui 8 globalement concave et la surface articulaire 2A globalement convexe correspondante du premier os 2. De façon préférentielle, la deuxième surface d'appui 8 est préférentiellement lisse de manière à favoriser encore davantage la mobilité de l'articulation 5. Avantageusement, le rétablissement du caractère régulier du mouvement (sans à-coup ni friction excessive) peut faciliter la régénération des tissus lésés, notamment cartilagineux, au niveau de la surface articulaire 2A.

Les moyens d'immobilisation 6, disposés sur la première surface d'appui 7, permettent ainsi d'immobiliser sensiblement l'implant 1 vis-à-vis du deuxième os 3, favorisant ainsi la régénération osseuse. La deuxième surface d'appui 8 favorise au contraire la mobilité du premier os 2 par rapport à l'implant 1 d'une part et au deuxième os 3 d'autre part.

Les moyens d'immobilisation 6 peuvent cependant être disposés sur la deuxième surface d'appui 8, globalement concave, et ce sans sortir du cadre de l'invention. Dans ce cas, la première surface d'appui 7 présente un état de surface sensiblement lisse, pour permettre la mobilité du deuxième os 3, par exemple la phalange, par rapport à l'implant 1.

Selon l'invention telle que illustré notamment sur la figure 3, les moyens d'immobilisation 6 comportent une pluralité de faces sensiblement planes 10 situées sur la première surface d'appui 7. Tel que cela est illustré sur la figure 3, les faces planes 10 sont sensiblement inclinées par rapport à l'axe longitudinal X-X'. Les faces planes 10 rompteur ainsi la symétrie de révolution de l'implant 1.

Les moyens d'immobilisation 6 pourraient, sans sortir du cadre de l'invention, également comporter une ou plusieurs faces de courbure inversée par rapport à la courbure globale de la première surface d'appui 7. Ces faces pourraient ainsi, dans le cas d'une première surface d'appui 7 globalement convexe, être formées par des faces creuses (ou concaves).

Avantageusement, les faces planes 10 présentent des aspérités (non représentées), et par exemple des stries facilitant l'accrochage et l'adhérence de l'implant 1 sur la surface articulaire 3A située en regard de la première surface d'appui 7, et donc son immobilisation en rotation relativement à ladite surface articulaire 3A. L'aspect rugueux de la première surface d'appui 7 est également de nature à favoriser la régénération osseuse du côté du deuxième os 3, c'est-à-dire, dans le cas d'une articulation métatarso-phalangienne, du côté phalangien.

Tel que cela est illustré sur la figure 3, les moyens d'immobilisation 6 comportent une pluralité de faces planes 10 situées sur la première surface d'appui 7, et sensiblement inclinées les unes par rapport aux autres de manière à former un polyèdre d'axe principal Y-Y'.

Au sens de l'invention, l'«*axe principal*» Y-Y' du polyèdre s'étend entre le sommet S et la base B fictive du polyèdre (illustrée en pointillés sur la figure 7), et de façon sensiblement perpendiculaire à ladite base B.

Avantageusement, chaque face plane 10 de l'implant 1 est inclinée selon un angle α compris entre 45° et 75°, et préférentiellement de l'ordre de 60° par rapport à la l'axe principal Y-Y' du polyèdre.

Tel que cela est illustré sur la figure 6, l'angle β au sommet S du polyèdre a une valeur comprise entre 90° et 150°, préférentiellement de l'ordre de 120°.

Les faces planes 10 sont préférentiellement juxtaposées de manière à présenter, deux à deux, au moins un côté commun 11. Selon une variante de réalisation illustrée sur la figure 4, l'implant 1 comporte, entre deux faces planes 10 successives, un bourrelet 12 de raccordement. Il est toutefois bien évidemment envisageable de réaliser un implant 1 dans lequel les faces planes 10 soient raccordées les unes aux autres sans sur-épaisseur, et donc sans bourrelet de raccordement.

Selon une variante de réalisation encore plus préférentielle de l'invention, les moyens d'immobilisation 6 sont formés par quatre faces planes 10 juxtaposées et disposées en forme de pyramide (figure 3).

Cette forme particulière de l'implant 1 lui permet de pénétrer sensiblement et efficacement au sein du creux formé par la surface articulaire 3A concave.

Avantageusement, la première surface d'appui 7 de l'implant 1 est formée par une combinaison de faces planes 10 et de faces courbes 13 orientées dans l'espace les unes par rapport aux autres de manière à conférer à la première surface d'appui 7 une forme globalement convexe. La première surface d'appui 7 présente ainsi une pluralité de discontinuités dans sa courbure, en particulier dans un plan P sensiblement médian et perpendiculaire à l'axe longnudinal X-X de l'implant 1.

Selon une variante de réalisation illustrée sur la figure 6, l'axe principal Y-Y' du polyèdre est sensiblement confondu avec l'axe longitudinal X-X' de l'implant 1, qui correspond également à l'axe de symétrie de révolution de la deuxième surface d'appui 8 concave.

Selon une autre variante de réalisation illustrée sur la figure 7, l'axe principal Y-Y' du polyèdre est sensiblement incliné selon un angle γ par rapport à l'axe longitudinal X-X'. De façon préférentielle, l'axe principal Y-Y' du polyèdre est incliné d'un angle γ compris entre 5° et 25° par rapport à l'axe longitudinal X-X'.

L'inclinaison du polyèdre permet ainsi de reproduire la morphologie de certaines articulations, notamment les articulations métatarso-phalangiennes, pour lesquelles les os, c'est-à-dire le métatarsien et la phalange, ne sont pas nécessairement alignés mais légèrement inclinés l'un par rapport à l'autre. Ainsi, un implant 1 destiné à être positionné entre une phalange et un métatarsien du pied présentera avantageusement un dimensionnement particulier.

L'implant 1 métatarso-phalangien conforme à l'invention présente ainsi, de préférence, une épaisseur moyenne de l'ordre de 4 mm mais peut également comporter des variations d'épaisseur de manière à reproduire la dorsiflexion de l'articulation 5. L'implant 1 peut ainsi se décomposer en plusieurs secteurs angulaires d'épaisseurs différentes. Dans le cas où l'implant 1 est dimensionné pour être positionné entre une phalange et un métatarsien du pied, il se décompose préférentiellement en un secteur plantaire SP, situé du côté de la plante du pied lorsqu'il est mis en place au sein de l'articulation 5, et un secteur dorsal SD, situé du côté de la partie dorsale du pied lorsque l'implant 1 est mis en place, le secteur plantaire SP présentant une épaisseur sensiblement supérieure à celle du secteur dorsal SD, de manière à reproduire l'inclinaison naturelle du métatarsien et de la phalange.

De façon particulièrement avantageuse, le rapport d'épaisseur entre le secteur plantaire et le secteur dorsal est compris entre 1,5 et 2,5.

L'implant 1 conforme à l'invention est en outre avantageusement dimensionné de telle manière qu'il puisse être glissé avec un faible jeu entre les surfaces articulaires 2A, 3A, de telle sorte qu'une fois positionné dans l'interface articulaire 4, l'implant 1 ne soit pas situé à distance des surfaces articulaires 2A, 3A mais au contact de ces dernières.

Le diamètre de l'implant 1 est également ajusté au diamètre des os 2, 3 formant l'articulation, de telle sorte qu'il ne fasse, dans le pire des cas, que très légèrement saillie à l'extérieur de l'articulation 5.

Selon une autre caractéristique particulièrement avantageuse de l'implant 1 conforme à l'invention, et qui constitue d'ailleurs une invention à part entière, l'implant 1 comporte des moyens d'auto-centrage 14 conformés pour venir en appui contre au moins l'une des surfaces articulaires 2A, 3A, assurant ainsi le centrage diamétral automatique de l'implant 1 vis-à-vis des os 2, 3.

Les moyens d'auto-centrage sont ainsi avantageusement conformés pour faire sensiblement coïncider l'axe longitudinal X-X' central de l'implant 1 avec les axes des canaux médullaires des premier et deuxième os 2, 3.

Avantageusement, les moyens d'auto-centrage 14 sont préférentiellement disposés sur la première surface d'appui 7, globalement convexe, de l'implant 1 de manière à venir en appui sur la surface articulaire 3A, sensiblement concave, du deuxième os 3.

Plus précisément, les moyens d'auto-centrage 14 sont avantageusement conformés pour venir en appui sensiblement ponctuel sur la surface articulaire 3A située en regard, et pour se positionner naturellement, dans une position d'équilibre stable, dans la partie la plus profonde de la surface articulaire 3A.

A cet effet, les moyens d'auto-centrage 14 sont préférentiellement pourvus d'au moins une protubérance 15, telle qu'un mamelon, faisant saillie sensiblement au centre de l'implant à partir de la première surface d'appui 7 globalement convexe. La protubérance 15 est ainsi sensiblement alignée avec l'axe longitudinal X-X' central, et s'étend sensiblement dans la même direction que ce dernier.

Contrairement aux implants de l'art antérieur, l'implant 1 conforme à l'invention est donc dépourvu d'orifice central et se présente avantageusement sous la forme d'un implant sensiblement plein et monobloc.

Selon une variante préférentielle de réalisation de l'invention illustrée sur la figure 8, la protubérance 15 et les faces planes 10 sont avantageusement disposées en quinconce, la protubérance 15 étant située sensiblement au centre de l'implant 1, et les faces planes 10 en périphérie de l'implant 1.

Tel que cela est illustré sur les figures 8 et 9, les première et deuxième surfaces d'appui 7, 8 se rejoignent au niveau d'une ligne frontière 16 qui, suivant le cas, peut être circulaire (figure 8) ou polygonale (figure 9).

De façon préférentielle, l'implant 1 articulaire d'interposition est fabriqué, au moins partiellement, en pyrocarbone de manière à favoriser la régénération des tissus osseux. Le pyrocarbone permet ainsi, grâce à ses propriétés mécaniques sensiblement identiques à celles des tissus osseux, d'obtenir de faibles couples de friction entre l'implant 1 et les os 2, 3 formant l'articulation, limitant ainsi le phénomène de lyse osseuse.

Toutefois, il est envisageable d'utiliser pour la fabrication de l'implant un autre matériau bio-compatible dont les propriétés mécaniques seraient adaptées à l'application décrite, notamment des céramiques ou des matériaux métalliques.

La méthode de pose de l'implant 1 conforme à l'invention va maintenant être décrite en se référant aux figures 1 à 9. Cette méthode est décrite à titre illlustratif et ne fait pas partie de l'invention revendiquée.

La méthode chirurgicale de pose de l'implant 1 conforme à l'invention comporte tout d'abord une étape d'incision, au cours de laquelle on réalise une incision de dimensions au moins égales, et à peine supérieures à celles de l'implant 1 au niveau de l'articulation 5 de manière à permettre l'introduction de l'implant 1. Suivant le degré d'altération de l'articulation 5, le chirurgien peut éliminer ou non les irrégularités des surfaces articulaires 2A, 3A en regard, de manière à leur conférer une forme sensiblement plus lisse et arrondie.

La méthode chirurgicale d'arthroplastie comporte ensuite une étape (a) d'immobilisation de l'implant 1 articulaire d'interposition entre les surfaces articulaires 2A, 3A des os 2, 3, au cours de laquelle on vient directement positionner l'implant 1 dans l'interface articulaire 4 de telle sorte qu'il vienne en appui, et s'immobilise par le biais de ses propres moyens d'immobilisation 6, contre au moins l'une des surfaces articulaires 2A, 3A.

Ainsi, on immobilise l'implant sans recourir à aucun accessoire, ni organe de fixation, ni aucun moyen d'immobilisation rapporté, tel que, par exemple, une broche d'immobilisation ou de fixation.

La méthode comporte en outre, avantageusement, une étape (b) d'auto-centrage de l'implant 1 au cours de laquelle on vient directement positionner l'implant 1 dans l'interface articulaire 4 de telle sorte qu'il vienne en appui, par le biais de ses propres moyens d'auto-centrage 14, contre au moins l'une des surfaces articulaires 2A, 3A, de manière à se centrer lui-même spontanément.

Ainsi, on réalise le centrage de l'implant intuitivement et immédiatement, sans avoir à recourir à aucun moyen de positionnement ni à aucun dispositif accessoire de centrage, tel que, par exemple, une broche de centrage.

De façon particulièrement avantageuse, les étapes (a) d'immobilisation et (b) d'auto-centrage de l'implant 1 s'effectuent de façon simultanée.

Par ailleurs, lors de l'étape (b) d'auto-centrage, on vient positionner les moyens d'auto-centrage 14 de l'implant 1 en appui contre la surface articulaire 3A sensiblement concave de l'un des os 2, 3, de telle sorte que lesdits moyens d'auto-centrage 14 se positionnent naturellement dans la partie la plus profonde de la surface articulaire 3A. De cette façon, la protubérance 15 et les faces planes 10 viennent sensiblement simultanément en appui contre la surface articulaire 3A, assurant ainsi simultanément d'une part le centrage diamétral de l'implant 1 au sein de l'interface articulaire 4 et d'autre part son immobilisation en rotation, grâce notamment à l'adhérence des faces planes 10 sur la surface articulaire 3A.

Avantageusement, après les étapes (a) d'immobilisation et (b) d'auto-centrage, on laisse reposer l'implant 1 dans l'interface articulaire 4, sans le fixer.

La méthode chirurgicale ici décrite permet donc, à l'aide d'un seul geste, de centrer et d'immobiliser l'implant 1 au sein de l'interface articulaire 4, supprimant ainsi les étapes classiques supplémentaires de centrage et de fixation de l'implant à l'aide d'une broche.

La mise en place de l'implant 1 articulaire d'interposition conforme à l'invention ne nécessite avantageusement aucune préparation préalable des surfaces articulaires, et ne requiert en particulier aucune opération de fraisage, grâce notamment aux première et deuxième surfaces d'appui globalement courbes de l'implant 1.

Un autre avantage de l'implant 1 conforme à l'invention est qu'il permet, grâce à la nature des matériaux le constituant mais également à sa forme anatomique et aux moyens d'immobilisation 6 intégrés structurellement à l'implant 1, de limiter le phénomène de lyse osseuse.

Un autre avantage de l'implant 1 conforme à l'invention est que sa pose est rapide et s'effectue en un nombre minimum d'étapes, ce qui permet de limiter le risque de complications et d'erreurs opératoires.

## Revendications

1. Implant articulaire d'interposition destiné à être mis en place entre les surfaces articulaires (2A, 3A) d'au moins deux os (2, 3) séparés par une interface articulaire (4) en vue de réaliser l'arthroplastie d'une articulation (5), ledit implant (1) étant **caractérisé en ce qu'**il s'étend suivant un axe longitudinal (X-X') et **en ce qu'**il comporte des moyens d'immobilisation (6) qui comportent une pluralité de faces sensiblement planes (10) disposées sur une première surface d'appui (7) globalement courbe formant une Interface de contact avec l'une des surfaces articulaires (2A, 3A), et sensiblement inclinées les unes par rapport aux autres de manière à former un polyèdre, de telle manière que, lorsque ledit implant est disposé dans l'interface articulaire (4), lesdits moyens d'immobilisation (6) viennent en appui contre au moins l'une des surfaces articulaires (2A, 3A) pour empêcher la rotation dudit implant (1) sur lui-même par rapport audit axe longitudinal (X-X').

2. Implant selon la revendication 1 **caractérisé en ce que** les moyens d'immobilisation (6) sont solidaires de l'implant (1).

3. Implant selon la revendication 1 ou 2 **caractérisé en ce qu'**il comporte au moins une première surface d'appui (7) globalement courbe formant une interface de contact avec l'une des surfaces articulaires (2A, 3A), les moyens d'immobilisation (6) étant disposés sur ladite première surface d'appui (7) de telle manière que l'intersection entre ladite première surface d'appui (7) et au moins un plan (P) perpendiculaire à l'axe longitudinal (X-X') forme une ligne de courbure discontinue.

4. Implant selon la revendication 3 **caractérisé en ce que** les moyens d'immobilisation (6) comportent au moins une face sensiblement plane (10) située sur ladite première surface d'appui (7).

5. Implant selon la revendication 4 **caractérisé en ce que** ladite face plane (10) est sensiblement inclinée par rapport à l'axe longitudinal (X-X').

6. Implant selon la revendication 4 ou 5 **caractérisé en ce que** ladite face plane (10) présente des aspérités.

7. Implant selon l'une des revendications 3 à 6 **caractérisé en ce qu'**il comporte une première et une deuxième surfaces d'appui (7, 8) globalement courbes et sensiblement parallèles l'une par rapport à l'autre.

8. Implant selon la revendication 7 **caractérisé en ce que** la première surface d'appui (7) est globalement convexe et **en ce que** la deuxième surface d'appui (8) est globalement concave.

9. Implant selon l'une des revendications précédentes **caractérisé en ce qu'**il comporte une deuxième surface d'appui (8) sensiblement sphérique.

10. Implant selon l'une des revendications précédentes **caractérisé en ce que** l'angle (β) au sommet (S) du polyèdre a une valeur comprise entre 90° et 150°, préférentiellement de l'ordre de 120°.

11. Implant selon l'une des revendications précédentes **caractérisé en ce que** lesdites faces planes (10) sont juxtaposées de manière à présenter, deux à deux, au moins un côté commun (11).

12. Implant selon la revendication 11 **caractérisé en ce qu'**il comporte, entre deux faces planes (10) successives, un bourrelet de raccordement (12).

13. Implant selon l'une des revendications précédentes **caractérisé en ce que** les moyens d'immobilisation (6) sont formés par quatre faces planes (10) juxtaposées et disposées en forme de pyramide.

14. Implant selon l'une des revendications précédentes **caractérisé en ce qu'**il comporte des moyens d'auto-centrage (14), conformés pour venir en appui contre au moins l'une des surfaces articulaires (2A, 3A), assurant ainsi le centrage automatique de l'implant (1) vis-à-vis des os (2. 3).

15. Implant selon la revendication 14 **caractérisé en ce que** les moyens d'auto-centrage (14) sont disposés sur la première surface d'appui (7).

16. Implant selon l'une des revendications 14 ou 15 **caractérisé en ce que** les moyens d'auto-centrage (14) sont pourvus d'au moins une protubérance (15), faisant saillie sensiblement au centre de l'implant (1).

17. Implant selon les revendications 13 et 18 **caractérisé en ce que** la protubérance (15) et les faces planes (10) sont disposées en quinconce, la protubérance (15) étant située sensiblement au centre de l'implant (1), et les faces planes (10) en périphérie de l'implant (1).

18. Implant selon l'une des revendications précédentes **caractérisé en ce que** l'axe principal (Y-Y') du polyèdre est sensiblement confondu avec l'axe longitudinal (X-X').

19. Implant selon l'une des revendications 1 à 17 **caractérisé en ce que** l'axe principal (Y-Y') du polyèdre est sensiblement incliné par rapport à l'axe longitudinal (X-X').

20. Implant selon la revendication .19 **caractérisé en ce que** l'axe principal (Y-Y') du polyèdre est incliné d'un angle (a) compris entre 5° et 25° par rapport à l'axe longitudinal (X-X').

21. Implant selon l'une des revendications précédentes **caractérisé en ce que** la première surface d'appui (7) de l'implant (1) est formée par une combinaison de faces planes (10) et de faces courbes (13), orientées dans l'espace de manière à conférer à ladite première surface d'appui (7) une forme globalement convexe.

22. Implant selon l'une des revendications précédentes **caractérisé en ce qu'**il est, au moins partiellement, en pyrocarbone.

23. Implant selon l'une des revendications précédentes **caractérisé en ce qu'**il se décompose en un secteur plantaire (SP) et un secteur dorsal (SD), le secteur plantaire (SP) présentant une épaisseur sensiblement supérieure à celle du secteur dorsal (SD).

24. Implant selon l'une des revendications précédentes **caractérisé en ce qu'**il est sensiblement plein et **en ce qu'**il constitue un implant (1) monobloc.

25. Implant selon l'une des revendications précédentes **caractérisé en ce qu'**il est dimensionné pour être positionné entre une phalange et un métatarsien du pied ou de la main, et constitue ainsi un implant (1) d'interposition métatarso-phalangien.

26. Implant selon l'une des revendications précédentes **caractérisé en ce que** les moyens d'immobilisation (6) sont conformés pour venir naturellement en appui contre la surface articulaire (3A).

## Claims

1. Articular interposition implant designed to be put in place between the articular surfaces (2A, 3A) of at least two bones (2, 3) separated by an articular interface (4) for the purpose of achieving arthroplasty of a joint (5), said implant(1) being **characterized in that** it extends along a longitudinal axis (X-X') and **in that** it comprises immobilisation means (6) which comprise a plurality of essentially plane faces (10) situated on a first support surface (7) on the whole curved forming a contact interface with one of the articular surfaces (2A, 3A), and substantially inclined with respect to each other in such a way as to form a polyhedron, so that when said implant is arranged in the articular interface (4), the immobilisation means (6) are supportive of at least one of the articular surfaces (2A, 3A), to prevent rotation of said implant (1) on itself with respect to said longitudinal axis (X-X').

2. Implant as claimed in claim 1, **characterised in that** the immobilisation means (6) are of one piece with the implant (1).

3. Implant as claimed in claim 1 or 2, **characterised in that** it comprises at least a first support surface (7), on the whole curved, forming a contact interface with one of the articular surfaces (2A, 3A), immobilisation means (6) being arranged on said first support surface (7) in such a way that the intersection between said first support surface (7) and at least one plane (P) perpendicular to the longitudinal axis (X-X') forms a line of discontinuous curvature.

4. Implant as claimed in claim 3, **characterised in that** immobilisation means (6) comprise at least one essentially plane surface (10) situated on said first support surface (7).

5. Implant as claimed in claim 4, **characterised in that** said plane surface (10) is substantially inclined with respect to longitudinal axis (X-X').

6. Implant as claimed in claim 4 or 5, **characterised in that** said plane surface (10) presents asperities, for example striae.

7. Implant as claimed in one of claims 3 to 6, **characterised in that** it comprises a first and a second support surface (7, 8), on the whole curved and essentially parallel with respect to each other.

8. Implant as claimed in claim 7, **characterised in that** the first support surface (7) is on the whole convex and **in that** the second support surface (8) is on the whole concave.

9. Implant as claimed in any of the preceding claims, **characterised in that** it comprises a second support surface (8) essentially spherical.

10. Implant as claimed in any of the preceding claims, **characterised in that** the angle (β) at the apex (S) of the polyhedron has a value between 90° and 150°, preferentially on the order of 120°.

11. Implant as claimed in any of the preceding claims, **characterised in that** said plane surfaces (10) are juxtaposed in such a way as to present, two by two, at least one common side (11).

12. Implant as claimed in claim 11, **characterised in that** it comprises, between two successive plane surfaces (10), a connecting flange (12).

13. Implant as claimed in one of the preceding claims, **characterised in that** the immobilisation means (6) are formed by four juxtaposed plane surfaces (10) arranged in the shape of a pyramid.

14. Implant as claimed in one of the preceding claims, **characterised in that** comprises self-centring means (14), shaped to be supportive of at least one of the articular surfaces (2A, 3A), thus ensuring automatic centring of implant (1) with respect to bones (2, 3).

15. Implant as claimed in claims 14, **characterised in that** the self-centring means (14) are arranged on the first support surface (7).

16. Implant as claimed in one of claims 14 or 15, **characterised in that** self-centring means (14) are provided with at least one protuberance (15), protruding essentially in the centre of implant (1).

17. Implant as claimed in claims 13 and 16, **characterised in that** the protuberance (15) and the plane surfaces (10) are arranged in a staggered fashion, the protuberance (15) being situated essentially in the centre of the implant (1), with the plane surfaces (10) on the periphery of the implant (1).

18. Implant as claimed in one of the preceding claims, **characterised in that** the principal axis (Y-Y') of the polyhedron is approximately coincident with the longitudinal axis (X-X').

19. Implant as claimed in one of claim 1 to 17, **characterised in that** the principal axis (Y-Y') of the polyhedron is substantially inclined with respect to longitudinal axis (X-X').

20. Implant as claimed in claim 19, **characterised in that** the principal axis (Y-Y') of the polyhedron is inclined by an angle (α) between 5° and 25° with respect to the longitudinal axis (X-X').

21. Implant as claimed in one of the preceding claims, **characterised in that** the first support surface (7) of implant (1) is formed by a combination of plane surfaces (10) and curved surfaces (13), oriented in space in such a way as to confer a shape that is on the whole convex on said first support surface (7).

22. Implant as claimed in one of the preceding claims, **characterised in that** it is, at least partially, made of pyrocarbon.

23. Implant as claimed in one of the preceding claims, **characterised in that** it breaks down into a plantar section (SP) and a dorsal section (SD), the plantar section (SP) presenting a thickness appreciably greater than that of the dorsal section (SD).

24. Implant as claimed in one of the preceding claims, **characterised in that** is essentially solid and **in that** it constitutes an implant (1) in one piece.

25. Implant as claimed in one of the preceding claims, **characterised in that** it is dimensioned to be positioned between a phalanx and a metatarsal of the foot or hand, and thus constitutes a metatarsal-phalangeal interposition implant (1).

26. Implant as claimed in one of the preceding claims, **characterised in that** immobilisation means (6) are shaped so as to be naturally supportive of articular surface (3A).

## Patentansprüche

1. Zwischenlegungsgelenkimplantat zum Einsetzen zwischen den Gelenkflächen (2A, 3A) von mindestens zwei Knochen (2, 3), die durch eine Gelenkschnittstelle (4) getrennt sind, um die Arthroplastik eines Gelenks (5) durchzuführen, wobei das Implantat (1) **dadurch gekennzeichnet ist, dass** es sich entlang einer Längsachse (X-X') erstreckt und dass es Festlegungsmittel (6) umfasst, die eine Vielzahl von im Wesentlichen flachen Seiten (10) umfassen, die auf einer ersten insgesamt gekrümmten Stützfläche (7) angeordnet sind, die eine Kontaktschnittstelle mit einer der Gelenkflächen (2A, 3A) bildet, und die im Wesentlichen zueinander geneigt sind, um ein Polyeder zu bilden, so dass sich, wenn das Implantat in der Gelenkschnittstelle (4) angeordnet ist, die Festlegungsmittel (6) an mindestens einer der Gelenkflächen (2A, 3A) abstützen, um die Drehung des Implantats (1) um sich selbst im Verhältnis zur Längsachse (X-X') zu verhindern.

2. Implantat nach Anspruch 1, **dadurch gekennzeichnet, dass** die Festlegungsmittel (6) mit dem Implantat (1) fest verbunden sind.

3. Implantat nach Anspruch 1 oder 2, **dadurch gekennzeichnet, dass** es mindestens eine erste insgesamt gekrümmte Stützfläche (7) umfasst, die eine Kontaktschnittstelle mit einer der Gelenkflächen (2A, 3A) bildet, wobei die Festlegungsmittel (6) auf der ersten Stützfläche (7) derart angeordnet sind, dass der Schnittpunkt zwischen der ersten Stützfläche (7) und mindestens einer zur Längsachse (X-X') rechtwinkligen Ebene (P) eine ununterbrochene Krümmungslinie bildet.

4. Implantat nach Anspruch 3, **dadurch gekennzeichnet, dass** die Festlegungsmittel (6) mindestens eine im Wesentlichen flache Seite (10) umfassen, die sich auf der ersten Stützfläche (7) befindet.

5. Implantat nach Anspruch 4, **dadurch gekennzeichnet, dass** die flache Seite (10) im Verhältnis zur Längsachse (X-X') im Wesentlichen geneigt ist.

6. Implantat nach Anspruch 4 oder 5, **dadurch gekennzeichnet, dass** die flache Seite (10) Unebenheiten aufweist.

7. Implantat nach einem der Ansprüche 3 bis 6, **dadurch gekennzeichnet, dass** es eine erste und eine zweite insgesamt gekrümmte Stützfläche (7, 8) umfasst, die zueinander im Wesentlichen parallel sind.

8. Implantat nach Anspruch 7, **dadurch gekennzeichnet, dass** die erste Stützfläche (7) insgesamt konvex ist und dass die zweite Stützfläche (8) insgesamt konkav ist.

9. Implantat nach einem der vorhergehenden Ansprüche, **dadurch gekennzeichnet, dass** es eine zweite im Wesentlichen sphärische Stützfläche (8) umfasst.

10. Implantat nach einem der vorhergehenden Ansprüche, **dadurch gekennzeichnet, dass** der Winkel (β) an der Spitze (S) des Polyeders einen Wert aufweist, der zwischen 90° und 150°, bevorzugt bei ungefähr 120° liegt.

11. Implantat nach einem der vorhergehenden Ansprüche, **dadurch gekennzeichnet, dass** die flachen Seiten (10) derart nebeneinander liegen, dass sie paarweise mindestens eine gemeinsame Seite (11) aufweisen.

12. Implantat nach Anspruch 11, **dadurch gekennzeichnet, dass** es zwischen zwei aufeinanderfolgenden flachen Seiten (10) eine Verbindungswulst (12) umfasst.

13. Implantat nach einem der vorhergehenden Ansprüche, **dadurch gekennzeichnet, dass** die Festlegungsmittel (6) aus vier flachen Seiten (10) gebildet sind, die nebeneinander liegen und pyramidenförmig angeordnet sind.

14. Implantat nach einem der vorhergehenden Ansprüche, **dadurch gekennzeichnet, dass** es selbstzentrierende Mittel (14) umfasst, die ausgebildet sind, um sich an mindestens einer der Gelenkflächen (2A, 3A) abzustützen, und somit die automatische Zentrierung des Implantats (1) gegenüber den Knochen (2, 3) sicherstellen.

15. Implantat nach Anspruch 14, **dadurch gekennzeichnet, dass** die selbstzentrierenden Mittel (14) auf der ersten Stützfläche (7) angeordnet sind.

16. Implantat nach einem der Ansprüche 14 oder 15, **dadurch gekennzeichnet, dass** die selbstzentrierenden Mittel (14) mit mindestens einer Ausstülpung (15) versehen sind, die im Wesentlichen in der Mitte des Implantats (1) vorsteht.

17. Implantat nach einem der Ansprüche 13 und 16, **dadurch gekennzeichnet, dass** die Ausstülpung (15) und die flachen Seiten (10) versetzt angeordnet sind, wobei sich die Ausstülpung (15) im Wesentlichen in der Mitte des Implantats (1) befindet und sich die flachen Seiten (10) am Umfang des Implantats (1) befinden.

18. Implantat nach einem der vorhergehenden Ansprüche, **dadurch gekennzeichnet, dass** die Hauptachse (Y-Y') des Polyeders im Wesentlichen mit der Längsachse (X-X') zusammenfällt.

19. Implantat nach einem der Ansprüche 1 bis 17, **dadurch gekennzeichnet, dass** die Hauptachse (Y-Y') des Polyeders im Verhältnis zur Längsachse (X-X') im Wesentlichen geneigt ist.

20. Implantat nach Anspruch 19, **dadurch gekennzeichnet, dass** die Hauptachse (Y-Y') des Polyeders im Verhältnis zur Längsachse (X-X) um einen Winkel (α) geneigt ist, der zwischen 5° und 25° liegt.

21. Implantat nach einem der vorhergehenden Ansprüche, **dadurch gekennzeichnet, dass** die erste Stützfläche (7) des Implantats (1) durch eine Kombination von flachen Seiten (10) und gekrümmten Seiten (13) gebildet ist, die derart räumlich orientiert sind, dass sie der ersten Stützfläche (7) eine insgesamt konvexe Form verleihen.

22. Implantat nach einem der vorhergehenden Ansprüche, **dadurch gekennzeichnet, dass** es mindestens teilweise aus Pyrokohlenstoff besteht.

23. Implantat nach einem der vorhergehenden Ansprüche, **dadurch gekennzeichnet, dass** es aus einem Sohlenteil (SP) und einem Rückenteil (SD) besteht, wobei das Sohlenteil (SP) eine Dicke aufweist, die im Wesentlichen größer ist als die des Rückenteils (SD).

24. Implantat nach einem der vorhergehenden Ansprüche, **dadurch gekennzeichnet, dass** es im Wesentlichen massiv ist und dass es ein einstückiges Implantat (1) bildet.

25. Implantat nach einem der vorhergehenden Ansprüche, **dadurch gekennzeichnet, dass** es dimensioniert ist, um zwischen einem Zehen- oder Fingerglied und einem mittelfuß- oder Mittelhandknochen positioniert zu sein, und somit ein Mittelfuß-Zehenglied- bzw. ein Mittelhand-Fingerglied-Zwischenlegungsimplantat (1) bildet.

26. Implantat nach einem der vorhergehenden Ansprüche, **dadurch gekennzeichnet, dass** die Festlegungsmittel (6) ausgebildet sind, um sich natürlich an der Gelenkfläche (3A) abzustützen.
